Europäisches Patentamt

European Patent Office

Office européen des brevets

(11) Publication number: **0 230 717**
**A1**

# EUROPEAN PATENT APPLICATION

(21) Application number: 86308334.1

(22) Date of filing: 27.10.86

(51) Int. Cl.⁴: **B01J 23/74** , B01J 37/03

(30) Priority: 18.12.85 GB 8531156

(43) Date of publication of application:
05.08.87 Bulletin 87/32

(84) Designated Contracting States:
BE DE FR IT NL

(71) Applicant: **British Gas plc**
**Rivermill House 152 Grosvenor Road**
**London SW1V 3JL(GB)**

(72) Inventor: **Banks, Reginald George Sinclair**
**55 St. Bernards Road Olton**
**Solihull West Midlands(GB)**
Inventor: **Oliver, James Alexander**
**54 Oeoffrey Road Shirley**
**Solihull West Midlands(GB)**

(54) Silica colloid catalyst.

(57) The mechanical stability of coprecipitated nickel-alumina catalysts used for methantion reactions, is improved by incorporating a silicon compound into the precursor prior to calcination of the precipitated form. Silicon, preferably in amounts of from 5 to 10% by weight, expressed at SiO₂, may be incorporated at the precipitation stage employing, for example, an alkali metal silicate or added to the precipitated form using, for example, colloidal silica.

EP 0 230 717 A1

This invention relates to catalysts, particularly methanation catalysts, to methods for their production and to the use of such catalysts.

In the production of methane-containing gases, for example by catalytically methanating synthesis gas, produced by the gasification of coal, severe reaction conditions may be encountered. Due to the high temperatures and pressures that are utilized and due to the presence of steam, conventional nickel-alumina methanation catalysts have an increasing tendency to sinter, with consequential loss of activity and mechanical strength.

Attempts to improve the mechanical strength of catalytic materials have included the use of organo-silicons as surface modifiers. For example in US Patent Specification No. 2722504 and in UK Patent Specification No. 1492274, there are disclosed techniques for impregnating metallic oxides having catalytic activity with silicones such as tetra-ethyl silicate, followed by heat treatment to decompose the organo-silicon to silica.

The use of organo silicones is itself a development of a process wherein the oxidic catalytic material was treated directly with silica, for example colloidal silica, this development arising out of the apparent ineffectiveness of the silica treatment. Thus, for example, as described in Example 9 of UK Patent Specification 1492274, an alumina substrate was impregnated with a colloidal silica, followed by drying and calcination. A comparison between this silica treated catalyst and those treated with organo-silicons purports to illustrate the superior properties of such organo-silicon treated catalysts.

Against this teaching of the prior art we have found that catalysts modified with silicon compounds such as colloidal silica can be produced which have all the advantages associated with organo-silicon modified catalysts and also have the following additional advantages:-

1. The need for an additional stage in the manufacture of the catalyst is avoided,

2. Inorganic silicon compounds such as colloidal silica are much cheaper than organo-silicon compounds,

3. Environmental problems and hazards associated with the handling of volatile organo-silicon compounds are avoided.

In accordance with the present invention there is provided an oxidic nickel-alumina catalyst precursor, which may be reduced to an active form wherein said precursor is formed by coprecipitation from a mixed solution of nickel and aluminium compounds and calcined to produce an oxidic precursor containing from 50 to 65% by weight of nickel, characterised in that a silicon compound is incorporated into the precursor prior to calcination to the oxidic form.

The present invention also provides a process for the production of oxidic catalyst precursors containing from 50 -65% by weight of nickel wherein the catalytic components are coprecipitated from a mixed aqueous solution of nickel and aluminium compounds and the thus formed precipitate is calcined to form a nickel -alumina precursor, characterised in that a silicon compound is incorporated prior to said calcination.

The general procedures for preparing the nickel -alumina precursors are described for example in UK Patent Specification Nos. 820257, 969637, 994278, 1150066, 1152009, 1155843, 1265481, 1525017, 1550749 and European Patent Publication No. 0010114.

The silicon compound, which may be an inorganic compound, may be added at any convenient stage during the catalyst preparation prior to calcination. Thus the silicon compound may be added as silica, e.g. as colloidal silica, to the washed and filtered precipitate prior to drying and calcination.

Alternatively, the silicon compound may be a water soluble compound and added, prior to precipitation, either to the mixed solutions of the catalyst component metals or to the precipitating agent. In this embodiment the silicon compound may be an alkali metal silicate. Where potassium ions are required for catalyst promotion it may be convenient to use potassium silicate as the additive and thus dispurse with a subsequent potassium addition stage.

However the silicon compound is added, it is preferred that it should be added in amounts sufficient that the final silicon content of the precursor is from 5 to 10% by weight, expressed as $SiO_2$, of the total calcined precursor.

The present invention will be further described and illustrated by the following Examples :

Example 1 (Comparison)

A catalyst precursor was prepared from nickel mitrate (31.8 kg), aluminium nitrate (15.9 kg) dissolved in 80 litres of water and sodium carbonate (24.4 kg), dissolved in 64 litres by the following method :-

The Preparation (Wet Stage)

The preparation was performed at constant temperature. The carbonate solution was heated to boiling and the nitrate solution to 92-93° then the carbonate was slowly run into the nitrate solution with vigorous stirring over a period of 35 minutes. The temperature of the nitrate solution was monitored by a thermocouple-digital readout unit and the steam supply to the steam jacket of the preparation vessel was adjusted to keep the temperature in the range 92-93°. At the end of the precipitation the slurry was held for a few minutes then heated to boiling and boiled gently for 30 minutes.

After boiling the slurry was passed to the rotary vacuum filter where it was filtered and washed on the filter using the spray bars. The cake was dripped into hot water and the resultant slurry made up to 180 litres and heated to 90° before the second filtration. The preparation was given six filtrations-an initial filtration and five wash filtrations on the final filtration ammonium carbonate (200 ppm) was added to the wash water and the spray bars were turned off to produce a dryer cake and the resulting cake was dropped into a steel tray where it was loaded into plastic bags for storage.

Working up of Catalyst

The preparation produced 44.6 kg of wet cake having a loss on drying of 70.6% and an overall loss on drying and calcination of 78.0%. The wet cake was placed in a fan oven and dried overnight at 125°C. The dried catalyst was calcined at 450°C for 2 hours and was then crushed to pass a 16 B.S. mesh sieve. It was blended with 2% graphite.

The final batch had the following composition and physical properties

Nickel content, % 56.6
Sodium content, % undetected
Potassium content, % undetected
Crush strength, kg 8.8
Bulk Density 0.97 g/ml
Water loss 11.2

Example 2 (Comparison)

This catalyst was prepared according to the manner described aforesaid in Example 1 then subsequently treated with tetra-ethyl silicate according to the teachings of UK Patent Specification No. 1492274. Thus, 650g of the pellets were vacuum impregnated with about 230 ml. of tetra-ethyl silicate, this volume being just enough to completely moisten the pellets. The pellets were heated in an oven in a stream of air at 100° for four hours to evaporate excess silicate and were then heated to 450°C for two hours to decompose the silicate. The resulting catalyst contained 1.3% silicon.

Example 3 (Comparison)

A catalyst was prepared as described aforesaid in Example 2, except that instead of tetra-ethyl silicate a colloidal silica sol was used.

For each 100g of starting catalyst 9 ml. of 'Syton X30FS colloidal silica' in 36 ml. of water was used, this amount being calculated to give 1.5% silicon in the catalyst. Electron microprobe analysis of the finished catalyst showed that very little of the silica had penetrated into the inside of the catalyst pellets.

Example 4

Following the procedure of Example 1, a catalyst precursor (wet stage) was obtained. However, prior to working up 933 gms of the wet catalyst cake (equivalent to 250 gms of calcined catalyst) were blended with 27 gms of Syton X30FS colloidal silica and sufficient water, about 250 ml., to form a smooth paste. The paste was then dried in a stream of air at 125° and worked up as described in Example 1.

Example 5

The procedure of Example 4 was repeated except that the amount of silica employed was doubled. Thus 54 gms of Syton X30FS colloidal silica was miced with about 250 ml of water and blended with 933 gms of catalyst cake.

Examples 6 -10

The procedure of Example 1 was followed except that potassium cilicate was added to the mixed nitrates solution prior to the precipitation stage. The amounts of silicate were sufficient to give the following weight percentage silica equivalents in the final catalyst :

| Example No. | %SiO$_2$ eqiv. |
|---|---|
| 6 | 1.0 |
| 7 | 2.0 |
| 8 | 5.0 |
| 9 | 7.5 |
| 10 | 10 |

Examples 11 -15

The procedures of Example 1 were followed except that potassium silicate was added to the carbonate solution prior to precipitation in sufficient amounts to give the following weight percentage silica equivalents in the final catalyst:

| Example No. | %SiO$_2$ Equiv |
|---|---|
| 11 | 1.0 |
| 12 | 2.0 |
| 13 | 5.0 |
| 14 | 7.5 |
| 15 | 10.0 |

High Temperature Sinter Test

The resistance of the catalysts to sintering was tested under simulated operating conditions by heating samples of the catalysts, after reduction in a stream of hydrogen, in a mixture of steam and hydrogen. Details are given in Table 1. After the test the samples were passivated in carbon dioxide at room temperature and their total surface areas were measured by nitrogen adsorption at the boiling point of liquid nitrogen, using the method of Brunauer, Emmett and Teller (J. American Chemical Society, 1938, 60 , 309). The results are given in Table 2.

**TABLE 1:  Sinter Test Conditions.**

Reduction

| | | |
|---|---|---|
| | Reductant | Hydrogen |
| | Temperature | 500°C |
| | Pressure | 25.2 bar |
| | Time | 16 hours |

Sinter Test

| | | |
|---|---|---|
| | Time | 270 hours |
| | Temperature | 550°C |
| | Pressure | 25.2 bar |
| | Steam/$H_2$ | 9/1 Molar |
| | Steam Flowrate | 102 ml hour$^{-1}$ water |
| | $H_2$ Flowrate | 0.015 m$^3$hr$^{-1}$ |

## Table 2: Surface Area of Catalyst after High Temperature Sinter Test

| Example No. | Total surface Area (BET) $m^2 \ g^1$ |
|:---:|:---:|
| 1 | 47 |
| 2 | 75 |
| 3 | 57 |
| 4 | 69 |
| 5 | 82 |
| 6 | 65 |
| 7 | 66 |
| 8 | |
| 9 | 82 |
| 10 | 80 |
| 11 | 67 |
| 12 | 69 |
| 13 | 84 |
| 14 | 88 |
| 15 | 94 |

It is apparent from a consideration of the results given in Table 2 that even at lower levels of silicon addition, catalysts of the invention exhibit surface area as good as the prior art organo-silicon post treated catalysts (Example 2) and markedly superior to those prior art catalysts post treated with silica.

## Hydrothermal Treatment Test

A catalyst of invention (Example 5), a prior art organo-silicon post-treated catalyst (Example 2) and an untreated control catalyst (Example 1) were treated for resistance to hydrothermal conditions by reducing it in a stream of hydrogen under the conditions given in the first part of Table 1. After reduction the catalyst was cooled to 300° and a steam-hydrogen mixture (9 $H_2O$ : 1 $H_2$ by moles) at 25.2 bar substituted for the pure hydrogen used for reduction. The temperature was then lowered to 220° at which temperature water condensed on the catalyst. This temperature was maintained for about 16 hours, then the temperature returned to 300° to evaporate any condensed water. After the steam/hydrogen mixture had been replaced by nitrogen the catalyst was cooled to room temperature and passivated in carbon dioxide.

Surface areas of the catalysts after hydrothermal treatment are listed in Table 3. It is clear that the catalysts of the invention have a much better resistance to hydrothermal treatment than the convention catalysts, and that such resistance is at least as good as that obtained by post-treating with organo-silicons as taught by the prior art.

TABLE 3: Surface Areas of Catalysts after Hydrothermal Treatment Test

| Catalyst | Total Surface Area (B.E.T.) $m^2 \ g^{-1}$ |
|---|---|
| 1 | 2 |
| 2 | 98 |
| 5 | 98 |

The catalysts of the invention have good mechanical stability and are suitable for use in the methanation of synthesis gas. Thus, for example, the catalysts of the invention may be employed in the process described and claimed in UK Patent Specification No. 1544245

Passivated catalysts which have been subjected to hydrothermal treatment exhibit, upon reactivation, improved sinter resistance as well as retaining catalytic activity. Hydrothermal treating can be employed in the preparative process. Thus according to a further embodiment of the invention catalysts prepared in accordance with the process of the invention can, following calcination, be first reduced, followed by contact with liquid water species at elevated temperatures, drying and further passivation.

Passivation may be effected with carbon dioxide according to known techniques, for example that described in UK Patent Specification No. 2118453.

**Claims**

1. An oxidic nickel-alumina catalyst precursor, which may be reduced to an active form wherein said precursor is formed by coprecipitation from a mixed solution of nickel and aluminium compounds and calcined to produce an oxidic precursor containing from 50 to 65% by weight of nickel characterised in that a silicon compound is incorporated into the precursor prior to calcination to the oxidic form.

2. A precursor as claimed in claim 1 wherein the silicon compound is an inorganic compound.

3. A percursor as claimed in claim 1 or claim 2 wherein the silicon content, expressed as silica in the calcined catalyst is from 5 to 10% by weight.

4. A reduced active form of a catalyst precursor as claimed in any one of the preceding claims.

5. A process for the production of oxidic catalyst precursors containing from 50 to 65% by weight nickel wherein the catalytic components are coprecipitated from a mixed aqueous solution of nickel and aluminium compounds and the thus formed precipitate is calcined to form a nickel-alumina precursor characterised in that a silicon compound is incorporated prior to said calcination.

6. A process as claimed in claim 5 wherein said silicon compound is an inorganic compound.

7. A process as claimed in claim 5 or claim 6 wherein said silica compound is a water soluble compound and is incorporated prior to coprecipitation.

8. A process as claimed in claim 7 wherein said silicon compound is an alkali metal silicate.

9. A process as claimed in claim 5 or claim 6 wherein the silicon is incorporated into the formed precipitate.

10. A process as claimed in claim 9 wherein the silica compound is colloidal silica.

11. A process as claimed in any one of claims 5 to 10 wherein the amount of silicon compound incorporated is such as to give a silicon content, expressed as silica, of from 5 to 10% by weight of the calcined oxidic precursor.

12. A process for the production of passivated nickel-alumina catalyst which comprises reducing an oxidic precursor produced by the process claimed in any one of claims 5 to 11 followed by contact with liquid water species at elevated temperature, drying and passivation.

13. A process for the production of nickel-alumina catalysts which comprises reducing an either an oxidic catalyst precursor produced by the process claimed in any one of claims 5 to 11 or passivated nickel-alumina catalysts.

14. Oxidic catalyst precursors as claimed in claim 1 and substantially as hereinbefore described.

15. A process for the production of oxidic catalyst precursors as claimed in claim 5 and substantially as hereinbefore described.

16. Passivated nickel-alumina catalysts whenever produced by the process as claimed in claim 12 and substantially as hereinbefore described.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 184 506 (RHONE-POULENC) | | B 01 J 23/74 |
| | | | B 01 J 37/03 |
| A | FR-A-2 325 427 (BRITISH GAS CORP.) | | |
| A | EP-A-0 029 675 (EXXON RESEARCH) | | |
| A | FR-A- 973 886 (LEVER BROTHERS & UNILEVER) | | |
| A | GB-A- 678 436 (N.V. DE BATAAFSCHE PETROLEUM MAATSCHAPPIJ) | | TECHNICAL FIELDS SEARCHED (Int. Cl.4) |
| A | US-A-3 324 195 (F.C.S. HWA) | | B 01 J 23/00 |
| | | | B 01 J 37/00 |

The present search report has been drawn up for all claims

| Place of search | Date of completion of the search | Examiner |
|---|---|---|
| THE HAGUE | 09-04-1987 | DEVISME F.R. |